Europäisches Patentamt

European Patent Office

Office européen des brevets

(11) Numéro de publication: **0 231 162 B1**

## (12) FASCICULE DE BREVET EUROPEEN

(45) Date de publication de fascicule du brevet:
**24.07.91**

(51) Int. Cl.⁵: **A61F 2/18**

(21) Numéro de dépôt: **87870012.9**

(22) Date de dépôt: **27.01.87**

(54) **Prothèse de l'oreille moyenne.**

(30) Priorité: **27.01.86 FR 8601073**

(43) Date de publication de la demande:
**05.08.87 Bulletin 87/32**

(45) Mention de la délivrance du brevet:
**24.07.91 Bulletin 91/30**

(84) Etats contractants désignés:
**BE DE GB IT NL**

(56) Documents cités:
**WO-A-83/03350**
**US-A- 4 130 905**
**US-A- 4 169 292**

(73) Titulaire: **Gersdorff, Michel**
**83 rue Vervloesem**
**B-1200 Bruxelles(BE)**

(72) Inventeur: **Gersdorff, Michel**
**83 rue Vervloesem**
**B-1200 Bruxelles(BE)**

(74) Mandataire: **Plucker, Guy et al**
**OFFICE KIRKPATRICK 4 Square de Meeûs**
**B-1040 Bruxelles(BE)**

## Description

La présente invention concerne une prothèse de l'oreille moyenne.

Le mécanisme naturel de transmission des vibrations sonores du tympan vers l'oreille interne se fait via la chaîne ossiculaire de l'oreille moyenne. Celle-ci est constituée de trois osselets articulés : le marteau, l'enclume et l'étrier, qui amplifient les vibrations du tympan pour transmettre le son à l'oreille interne. La fenêtre ovale, dans laquelle repose l'étrier (le plus petit des osselets), assure la communication entre l'oreille moyenne et l'oreille interne. L'ensemble constitué par le tympan et la chaîne des osselets est communément appelé "complexe tympano-ossiculaire".

De nombreuses pathologies de l'oreille moyenne (otites moyennes chroniques sous leurs différentes formes, traumatismes, séquelles d'interventions chirurgicales antérieures) peuvent entraîner comme conséquences une dysfonction du complexe tympano-ossiculaire. Cette dysfonction entraîne un trouble auditif sous forme d'une surdité de transmission. Il s'agit d'une altération de la fonction auditive caractérisée par la non-transmission correcte du message sonore se propageant de l'oreille externe vers l'oreille interne.

L'oreille moyenne fonctionne essentiellement comme un transformateur d'impédance acoustique. Si elle ne peut remplir ce rôle, il s'ensuit une diminution du signal d'entrée cochléaire et une surdité dite transmissionnelle.

Ces surdités de transmission sont principalement liées à une atteinte du tympan (perforation du tympan) ou à une lésion au niveau de la chaîne ossiculaire (ankylose ou lyse ossiculaire).

En l'absence totale du complexe tympano-ossiculaire, les ondes sonores transmises par le milieu aérien rencontreraient sans transition la platine de l'étrier, dans la fenêtre ovale de l'oreille interne, et seraient réfléchies.

Il en résulterait que seule une toute petite partie (de l'ordre d'un millième) de l'énergie sonore serait transmise au liquide de l'oreille interne.

Ceci entraînerait chez le patient une diminution de la sensation de l'intensité sonore perçue dans le labyrinthe de l'oreille interne. Cette diminution peut s'exprimer en décibels perdus.

Notons que la reconstruction des différentes parties osseuses de l'oreille moyenne ou externe peut se faire soit à l'aide d'allogreffe ossiculaire (osselet prélevé sur cadavre), soit à l'aide d'autogreffe ossiculaire (prélèvement d'un des osselets du patient, remodelage et rétablissement d'une continuité tympano-ossiculaire) ou, plus récemment, par mise en place d'une prothèse réalisée en un matériau dit biocompatible. Ces dernières années, on a utilisé particulièrement du polyéthylène poreux (tel que le polyéthylène poreux commercialisé sous la marque Proplast® ou des céramiques biocompatibles, notamment des céramiques biocompatibles bioactives (telles que celle commercialisée sous la marque Céravital®).

La reconstruction du mécanisme de la transmission du son dans l'oreille moyenne par implantation de prothèses biocompatibles est bien connue.

Il est en effet connu de remédier aux différents types de surdités de transmission en remplaçant différentes parties de l'oreille, en fonction de l'affection dont souffre le patient. On peut ainsi remplacer ou restaurer le cadre osseux de l'oreille externe, l'annulus entourant le tympan, la membrane tympanique elle-même, et chacun des trois osselets de la chaîne ossiculaire.

Selon les cas, et selon le type d'opération chirurgicale pratiqué, on peut distinguer différentes catégories de prothèses, pour la fabrication desquelles des matériaux variés peuvent être utilisés.

Dans le brevet US 4.169.292 de GROTE, est décrite une prothèse destinée à remplacer toute la structure de l'oreille depuis le canal osseux de l'oreille externe jusqu'à la fenêtre ovale, qui détermine la limite entre l'oreille moyenne et l'oreille interne.

La partie de la prothèse selon GROTE destinée à remplacer la chaîne ossiculaire est solidaire d'un anneau artificiel, lui-même relié à un tube remplaçant le canal osseux.

Ce type de prothèse ne peut être utilisé si seule la chaîne ossiculaire est défectueuse ou manquante.

Il est également connu de reconstruire seulement le complexe tympano-ossiculaire, ou seulement la chaîne ossiculaire de l'oreille moyenne, ou seulement une partie de celle-ci.

Une telle reconstruction peut se pratiquer en cas d'évidement radical. La prothèse est dans ce cas placée dans une position différente de celle qu'occupe naturellement la chaîne ossiculaire dans la cavité de l'oreille moyenne.

C'est le cas, par exemple, dans le brevet US 4.130.905, de MERCANDINO, qui décrit une prothèse consistant en une pièce unique remplaçant l'ensemble de la chaîne ossiculaire, la position du néotympan étant différente de la position normale du tympan.

L'utilisation d'une telle prothèse implique une opération chirurgicale modifiant totalement la structure de l'oreille moyenne.

Il est encore connu de reconstruire la chaîne ossiculaire de l'oreille moyenne lorsque la position du tympan (naturel ou néoformé) est conservée.

Il existe deux formes de prothèses de la chaîne ossiculaire. Lorsque l'étrier est intact, la prothèse, dite alors partielle, est appliquée entre le tympan (ou le néotympan) et le sommet de l'étrier. Lorsque seule la platine de l'étrier est conservée, la prothèse est dite totale et s'applique directement entre le tympan (ou le néotympan) et la platine.

Les prothèses totales actuellement utilisées sont généralement des prothèses qui fonctionnent sous forme de columelle, c'est-à-dire que l'on remplace l'ensemble de la chaîne ossiculaire (marteau-enclume-étrier), par une seule prothèse linéaire interposée directement entre le tympan (ou le néotympan) et la platine de l'étrier.

Des exemples de telles prothèses sont décrits dans les brevets US 3.909.852 et US 4.052.754 de HOMSY, dans le brevet US 4.287.616 de HEIMKE et al., et dans le brevet US 4.281.419 de TREACE.

Sur le plan physique, les prothèses en forme de columelle transmettent directement le son du tympan sur la platine de l'étrier et le tympan est en contact direct avec la prothèse.

Dans la chaîne ossiculaire normale, le marteau est plus long que l'enclume, et est articulé avec celle-ci par l'intermédiaire de l'articulation incudo-malléaire. Ceci a pour résultat que cette partie de la chaîne joue un rôle de bras de levier qui permet une amplification du son. Ce rôle n'est pas rempli par les prothèses en forme de columelle.

De plus, il existe dans la chaîne normale une seconde articulation, dite incudo-stapédienne, entre l'enclume et l'étrier.

Le rôle des articulations est très important puisqu'elles permettent à la fois une transmission douce et progressive du son et on obtient une amplification de celui-ci grâce au système du bras de levier. Ceci n'est pas le cas dans les prothèses à columelle.

Pour remédier aux inconvénients des prothèses totales linéaires utilisées jusqu'ici et pour rétablir les avantages qu'offre la chaîne ossiculaire normale dans le mécanisme de la transmission du son, le Demandeur a mis au point une prothèse articulée, qui assure une transmission non linéaire du son du tympan vers l'étrier. La prothèse selon l'invention peut être utilisée comme prothèse partielle ou totale, suivant que l'étrier est conservé intact ou que seule sa platine subsiste.

Elle peut encore être placée dans l'oreille, que les parties osseuses de celle-ci soient naturelles ou elles-mêmes reconstruites en tout ou en partie. De même, la prothèse selon l'invention peut fonctionner que le tympan soit intact ou remplacé par un néotympan.

La présente invention a donc pour objet une prothèse de l'oreille moyenne comportant un moyen de transmission du son entre la membrane tympanique et une partie constitutive de l'étrier,

ledit moyen de transmission comportant un premier bras et un second bras, plus court que le premier bras, caractérisé en ce que les deux bras, reliés entre eux à une de leurs extrémités par un élément de raccordement, sont orientés dans deux plans parallèles entre eux et sont aperçus formant entre eux un angle aigu lorsqu'ils sont vus suivant une direction perpendiculaire à ces plans,

l'élément de raccordement étant perpendiculaire à ces deux plans et reliant les bras aux extrémités qui formant le sommet de cet angle aigu,

le moyen de transmission étant relié à un moyen de fixation par un moyen de suspension qui est allongé, flexible, orientable et ajustable, et dont une extrémité est reliée au moyen de fixation, l'autre extrémité étant reliée au moyen de transmission du son en un point de l'extrémité de l'un de bras proche de l'élément de de raccordement entre les deux bras;

cette prothèse étant ainsi apte à être mise en place dans la cavité de l'oreille moyenne de manière telle que le moyen de fixation est inséré dans une partie osseuse de l'oreille moyenne, que le moyen de suspension est indépendant de la membrane tympanique, que le premier bras est en contact avec cette membrane tympanique, et que le second bras est en contact avec une partie constitutive de l'étrier.

L'extrémité libre du second bras peut être reliée à l'arche stapédienne de l'étrier par un deuxième élément de raccordement et forme entre le bras et l'arche un angle proche de l'angle droit.

Lorsque les branches de l'étrier ne sont pas conservées, mais qu'il persiste uniquement sa platine, ce qui arrive fréquemment, le second bras est relié à une tige ou columelle, dont l'extrémité libre assure le contact avec la platine de l'étrier. La columelle est donc reliée au second bras par un deuxième élément de raccordement, le second bras et la columelle formant entre eux un angle proche de l'angle droit.

Dans ce dernier cas, la prothèse selon l'invention est une prothèse totale.

Dans le cas où l'arche stapédienne de l'étrier est conservée, la prothèse selon l'invention est une prothèse partielle.

Le premier et/ou le deuxième élément de raccordement peuvent être soit fixes soit orientables et ajustables.

Dans la prothèse selon la présente invention, le tympan n'est en contact qu'avec le premier bras, qui représente le marteau. L'étrier, quand il est conservé intact, est en contact avec le second bras, qui est indépendant du premier. Lorsque seule la platine est conservée, la prothèse est en contact avec celle-ci par l'intermédiaire d'une columelle portée par l'extrémité du second bras.

De plus, la suspension de la prothèse ossiculaire à une partie osseuse de l'oreille moyenne peut se faire par insertion du moyen de fixation dans le cadre tympanique, dans le conduit auditif ou dans tout autre endroit de l'oreille moyenne selon la technique chirurgicale utilisée. Eventuellement, le moyen de fixation peut être inséré dans une partie de l'oreille artificiellement créée.

Le rôle du moyen de suspension de la prothèse est similaire à celui joué par les ligaments suspenseurs de la chaîne ossiculaire naturelle.

La prothèse selon l'invention permet la transmission douce des sons du tympan, vers la platine de l'étrier : le premier bras, qui représente le marteau, vibre avec le tympan, et le second bras, qui représente l'enclume, fait vibrer la platine de l'étrier.

La structure de la prothèse est telle qu'elle permet une suspension non rigide, souple, qui se rapproche des conditions physiologiques.

L'absence de contact entre la partie de la prothèse s'adaptant au tympan et celle s'adaptant à l'étrier (ou à sa platine) assure une diminution de l'impédance du système de transmission, grâce au principe du bras de levier.

Le fait que la prothèse soit suspendue à une partie osseuse de l'oreille, de manière indépendante de la membrane tympanique, assure un bon effet de compliance.

La partie de la prothèse de l'invention remplaçant les osselets ainsi que le plateau (moyen de fixation destiné à être fixé, par exemple, dans le cadre tympanique) sont fabriqués en matériau dit biocompatible, en particulier en céramique bioactive, par exemple une biocéramique vendue sous la marque Céravital®. Le moyen de suspension est orientable et ajustable et peut être réalisé en une matière synthétique biocompatible ou peut consister en un fil métallique, par exemple en acier inoxydable.

Afin d'assurer un contact optimum entre la columelle ou tige de la prothèse totale et la platine de l'étrier et pour maintenir cette tige au milieu de la platine, on place, de préférence sur la platine, un stabilisateur tel que celui décrit dans la demande FR-A-8601074 du même Demandeur déposée le 27 janvier 1986.

Lorsque la membrane tympanique est atteinte, la majorité des chirurgiens otologistes la remplacent actuellement par une greffe en un fragment d'aponévrose temporale. On peut également utiliser une allogreffe tympanique (à partir de tympan humain préservé) ou une xénogreffe tympanique (par exemple à partir d'une veine jugulaire de veau).

Dans le cas de remplacement de la membrane tympanique par un néotympan, la prothèse selon l'invention viendra s'appuyer contre celle-ci et jouera le même rôle que dans le cas d'une membrane naturelle.

De la même façon, lorsque la partie osseuse de l'oreille moyenne est endommagée, il est possible d'insérer le moyen de fixation de la prothèse selon l'invention dans une partie osseuse artificiellement créée.

A l'aide des figures en annexe, dans lesquelles les mêmes numéros réfèrent aux mêmes éléments, on décrit un exemple de réalisation de la prothèse selon l'invention.

La Fig. 1 est une vue schématique, partiellement en coupe de l'oreille (externe, moyenne et interne);

la Fig. 2 est une vue schématique de l'oreille moyenne, dans laquelle les osselets sont remplacés par une prothèse à columelle selon le brevet US 4.510.627;

la Fig. 3 est une vue schématique de l'oreille moyenne, dans laquelle les osselets sont remplacés par une prothèse totale selon la présente invention;

la Fig. 4 est une vue agrandie de la prothèse totale selon la présente invention avec son moyen de fixation et son moyen de suspension.

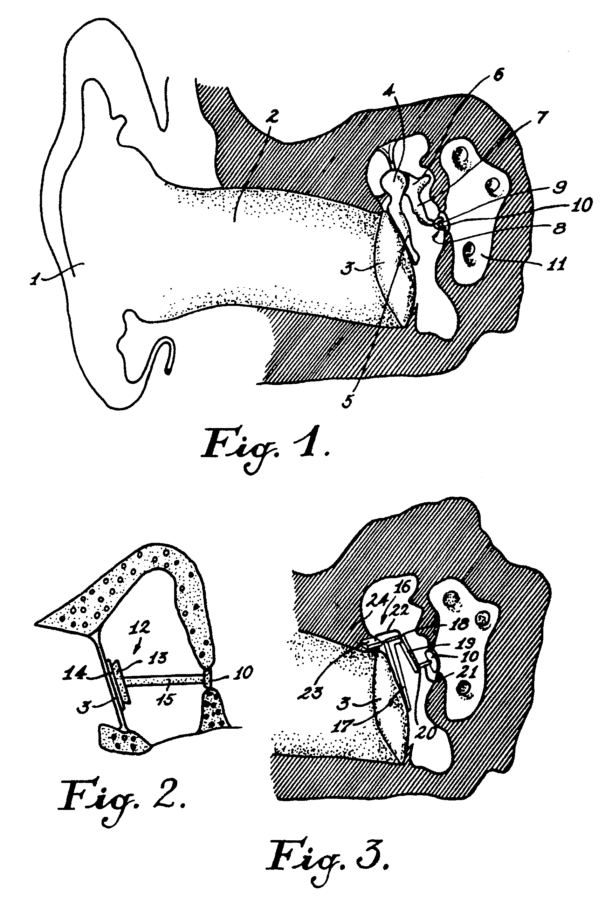

La Fig. 1 montre le pavillon de l'oreille externe 1, le conduit auditif externe 2, la membrane tympanique 3 et la chaîne ossiculaire de l'oreille moyenne constituée de ses trois osselets articulés : le marteau 4 avec sa longue apophyse (ou manche) 5, l'enclume 6 avec sa longue apophyse 7 et l'étrier 8 avec son arche stapédienne 9 et sa platine 10 et enfin l'oreille interne 11.

La Fig. 2 montre une prothèse totale 12 de l'oreille moyenne constituée d'une part d'une tête élargie ronde 13 adhérant à la membrane tympanique 3 au moyen d'un cartilage 14 et d'autre part d'une tige allongée (columelle) 15, s'appuyant sur la platine 10 de l'étrier 8.

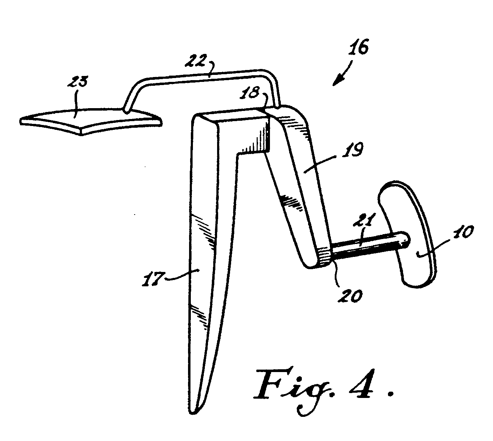

Les Fig. 3 et 4 montrent une prothèse totale 16 selon l'invention, comprenant un premier bras 17, représentant le marteau 4 et qui assure le contact avec la membrane tympanique 3. Le premier bras 17 est relié à angle aigu, à une extrémité d'un second bras 19, par l'élément de raccordement 18. Le second bras 19, représentant l'enclume 6, est relié à son autre extrémité, à un angle proche de l'angle droit, à une tige (columelle) 21 par le deuxième élément de raccordement 20. L'extrémité libre de la columelle 21 assure le contact avec la platine 10 de l'étrier 8. Il faut noter que le premier bras 17 est plus long que le second bras 19. Toute la prothèse 16 est suspendue au moyen du fil 22 en métal ou en tout autre matériau synthétique biocompatible, au plateau 23 fixé dans le cadre tympanique 24. Ce fil 22 est orientable et ajustable en sorte que, si le cadre tympanique 24 est endommagé, on peut fixer le plateau 23 au conduit auditif ou en toute autre partie naturelle ou artificielle de l'oreille moyenne, selon la technique chirurgicale utilisée. Les éléments de raccordement 18 et 20 peuvent également être orientables ou ajustables.

On comprend aisément que par rapport à la prothèse à columelle de la Fig. 2, la prothèse 16 selon l'invention (Fig. 3 et 4) offre des avantages indéniables.

Le bras 17 n'adhère pas de manière rigide et inséparable à la membrane tympanique 3. Il est fixé, par exemple, au cadre tympanique 24 et ce, moyennant une suspension souple, le fil 22 en métal (ou en un autre matériau biocompatible), tout comme le marteau 4 d'une oreille naturelle est suspendu dans la cavité de l'oreille moyenne au moyen de ligaments suspenseurs.

D'autre part, au lieu d'établir une liaison directe et rigide 15 entre la membrane tympanique 3 et la platine 10 de l'étrier, comme c'est le cas dans les prothèses connues 12, la prothèse 16 de l'invention comporte deux éléments de raccordement 18 et 20 séparant ainsi le contact avec la membrane tympanique 3 du contact avec la platine 10. Ceci assure une transmission plus physiologique des vibrations tympaniques à l'étrier 8 et, de là, à l'oreille interne 11.

La présence dans l'oreille moyenne de la prothèse 16 permet la transmission de l'énergie sonore, de manière que celle-ci ne soit pas réfléchie par la platine 10 de l'étrier 8 au niveau de la fenêtre ovale, et qu'il y ait un minimum de perte de sensation d'intensité sonore.

La structure dans l'espace de la prothèse 16 remplit un rôle important dans la mesure où la forme et la position des centres de gravité du bras 17 remplaçant le marteau 4 et du bras 19 remplaçant l'enclume 6 jouent un rôle important dans la transmission du son.

Une dépression de la membrane tympanique 3 entraîne un enfoncement de la platine 10 dans les liquides péri-lymphatiques de l'oreille interne 11. Le son incident entraîne ainsi dans l'oreille moyenne une succession d'oscillations complexes de la prothèse 16, ce qui aboutit à une succession de coups exercés sur la platine 10 de l'étrier 8.

La prothèse 16 permet aussi un déphasage des ondes entre la fenêtre ovale et la fenêtre ronde, lequel est indispensable à la physiologie de la cochlée. En effet, grâce à sa masse et à sa résistance statique, la chaîne ossiculaire transmet à la fenêtre ovale une onde qui est déphasée par rapport à celle qui parvient à la fenêtre ronde.

La surface de la platine 10 étant en moyenne de 2,5 mm² et celle de la partie vibrante de la membrane tympanique 3 de 35 mm², il en découle que l'énergie en parcourant la chaîne ossiculaire est concentrée sur la platine 10, ce qui accroît la sensation d'intensité sonore d'environ 23 dB. En effet, $10 \log (35/2,5)^2 = 10 \log 14^2 = 20 \log 14 = 23$ dB.

Cette récupération partielle de sensation d'intensité sonore grâce à la présence du complexe tympano-ossiculaire existe dans l'oreille normale et peut également exister dans le cas de prothèses totales du type columelle, pour autant que le rapport des surfaces mises en contact soit conservé.

Une partie supplémentaire de sensation d'intensité sonore peut encore être récupérée au niveau de la fenêtre ovale grâce à la prothèse 16 selon l'invention. Celle-ci exerce une fonction de levier, grâce à laquelle la pression due au mouvement translationnel est plus grande au niveau de la platine 10 qu'au niveau de la membrane tympanique 3.

Ceci résulte du fait que le bras 17 qui assure le contact avec la membrane tympanique 3 est plus long que le bras 19 qui assure le contact avec l'étrier 8 (ou qui, par l'intermédiaire de la columelle 21, assure le contact avec la platine 10 de l'étrier 8). Le déplacement de l'extrémité du court bras 19 et donc également de la platine 10 de l'étrier 8 sera donc moins important que celui de l'extrémité du long bras 17, mais cette réduction de l'amplitude de déplacement s'accompagne d'une augmentation de pression.

On estime l'amplitude du déplacement de la platine 10 à $10^{-11}$ - $10^{-12}$ centimètres.

Grâce à la fonction de levier de la prothèse 16, la pression exercée sur la platine 10 est égale à 1,31 fois la pression exercée sur la membrane tympanique 3, ce qui a pour résultat la récupération de 3 dB supplémentaires. En effet, $10 \log (1,31/1)^2 = 20 \log 1,31 = 3$ dB.

Par conséquent, la prothèse 16 selon l'invention permet de récupérer environ 26 dB par rapport au cas où le complexe tympano-ossiculaire est absent, soit 3 dB de plus que les prothèses columellaires connues.

## Revendications

1. Prothèse (16) de l'oreille moyenne comportant un moyen de transmission du son entre la membrane tympanique (3) et une partie constitutive de l'étrier (8),

   ledit moyen de transmission comportant un premier bras (17) et un second bras (19) plus court que le premier bras (17), caractérisée en ce que les deux bras (17, 19), reliés entre eux à une de leurs extrémités par un élément de raccordement (18), sont orientés dans deux plans parallèles entre eux et sont aperçus formant entre eux un angle aigu lorsqu'ils sont vus suivant une direction perpendiculaire à ces plans, l'élément de raccordement (18) étant perpendiculaire à ces deux plans et reliant les bras (17,19) aux extrémités qui forment le sommet de de cet angle aigu

   le moyen de transmission étant relié à un moyen de fixation (23) par un moyen de suspension (22) qui est allongé, flexible, orientable et ajustable, et dont une extrémité est reliée au moyen de fixation (23), l'autre extrémité étant reliée au moyen de transmission du son en un point de l'extrémité de l'un de bras (17,19) proche de l'élément de raccordement (18) entre les deux bras (17, 19);

cette prothèse (16) étant ainsi apte à être mise en place dans la cavité de l'oreille moyenne de manière telle que le moyen de fixation (23) est inséré dans une partie osseuse de l'oreille moyenne, que le moyen de suspension (22) est indépendant de la membrane tympanique (3), que le premier bras (17) est en contact avec cette membrane tympanique (3), et que le second bras (19) est en contact avec une partie constitutive de l'étrier (8).

2. Prothèse suivant la revendication 1, caractérisée en ce qu'elle est apte à être mise en place dans la cavité de l'oreille moyenne, de manière telle que le moyen de fixation (23) est inséré dans le conduit auditif.

3. Prothèse suivant la revendication 1, caractérisée en ce qu'elle est apte à être mise en place dans la cavité de l'oreille moyenne, de manière telle que le moyen de fixation (23) est inséré dans le cadre tympanique (24).

4. Prothèse suivant l'une quelconque des revendications 1 à 3, caractérisée en ce que l'élément de raccordement (18) est fixe.

5. Prothèse suivant l'une quelconque des revendications 1 à 3, caractérisée en ce que l'élément de raccordement (18) est orientable et ajustable.

6. Prothèse suivant l'une quelconque des revendications 1 à 5, caractérisée en ce qu'elle est apte à être mise en place dans la cavité de l'oreille moyenne, de manière telle que l'extrémité libre de son second bras (19) est reliée à l'arche stapédienne (9) de l'étrier (8) par un deuxième élément de raccordement (20) en formant entre le second bras (19) et l'arche (9) un angle proche de l'angle droit.

7. Prothèse suivant l'une quelconque des revendications 1 à 5, caractérisée en ce qu'elle comporte, en outre, une columelle (21) reliée au second bras (19) par un deuxième élément de raccordement formant avec le second bras (19) un angle proche de l'angle droit, cette columelle (21) étant apte à assurer, par son extrémité libre, le contact avec la platine (10)

de l'étrier (8), lorsque la prothèse (16) est mise en place dans la cavité de l'oreille moyenne.

8. Prothèse suivant l'une quelconque des revendications 6 et 7, caractérisée en ce que le deuxième élément de raccordement (20) est fixe.

9. Prothèse suivant l'une quelconque des revendications 6 et 7, caractérisée en ce que le deuxième élément de raccordement (20) est orientable et ajustable.

10. Prothèse suivant l'une quelconque des revendications précédentes, caractérisée en ce que le moyen de fixation (23) à une partie osseuse de l'oreille moyenne et le moyen de transmission du son sont en céramique biocompatible.

11. Prothèse suivant la revendication 10, caractérisée en ce que le moyen de fixation (23) à une partie osseuse de l'oreille moyenne et le moyen de transmission du son sont en céramique biocompatible bioactive.

12. Prothèse suivant l'une quelconque des revendications précédentes, caractérisée en ce que le moyen de suspension (22) est réalisé en une matière synthétique biocompatible.

13. Prothèse suivant l'une quelconque des revendications 1 à 11, caractérisée en ce que le moyen de suspension (22) consiste en un fil de métal.

**Claims**

1. A middle-ear prosthesis (16) comprising a sound transmission means between the tympanic membrane (3) and a constituent part of the stapes (8) said transmission means comprising a first arm (17), a second arm (19) shorter than the first arm (17), characterized in that

the two arms (17, 19) joined together by a connecting member (18) are directed in two planes parallel to one another and are seen to form an acute angle between them when viewed in a direction at right angles to said planes, the connecting member (18) being perpendicular to said two planes and connecting the two arms (17, 19) at their ends which form the top of said acute angle;

the transmission means being connected to a fastening means (23) by a suspension means (22) which is elongated, flexible, orientable and adjustable and of which one end is connected to the fastening means (23), the other end being connected to the sound transmission means at an end point of either of the arms (17, 19) close to the connecting member (18) between the two arms (17, 19);

said prosthesis (16) being implantable in the middle-ear cavity in such a way that the fastening means (23) is inserted into a bony part of the middle-ears, that the suspension means (22) is independent from the tympanic membrane (3), that the first arm (17) is in contact with said tympanic membrane (3), and that the second arm (19) is in contact with a constituent part of the stapes (8).

2. Prosthesis according to claim 1, characterized in that it is insertable in the middle-ear cavity in such a way that the fastening means (23) is inserted into the auditory meatus.

3. Prosthesis according to claim 1, characterized in that it is insertable in the middle-ear cavity in such a way that the fastening means (23) is inserted into the tympanic frame (24).

4. Prosthesis according to any one of claims 1 to 3, characterized in that the connecting member (18) is fixed.

5. Prosthesis according to any one of claims 1 to 3, characterized in that the connecting member (18) is orientable and adjustable.

6. Prosthesis according to any one of claims 1 to 5, characterized in that it is insertable in the middle-ear cavity in such a way that the free end of the second arm (19) is connected to the stapedial arch (9) of the stapes (8) by a second connecting member (20) by forming between the second arm (19) and the arch (9) an angle close to a right angle.

7. Prosthesis according to any one of claims 1 to 5, characterized in that it further comprises a columella (21) connected to the second arm (19) by a second connecting member forming with the second arm (19) an angle close to a right angle, said columella (21) being connectable by its free end to the base (10) of the stapes (8) when the prosthesis (16) is inserted in the middle-ear cavity.

8. Prosthesis according to any one of claims 6 and 7, characterized in that the second connecting member (20) is fixed.

9. Prosthesis according to any one of claims 6 and 7, characterized in that the second connecting member (20) is orientable and adjustable.

10. Prosthesis according to any one of the preceding claims, characterized in that the fastening means (23) adapted be fastened into a bony part of the middle-ear and the sound transmission means are of biocompatible ceramic material.

11. Prosthesis according to claim 10, characterized in that the fastening means (23) adapted to be fastened into a bony part of the middle-ear and the sound transmission means are of bioactive biocompatible ceramic material.

12. Prosthesis according to any one of the preceding claims, characterized in that the suspension means (22) is made of a biocompatible synthetic material.

13. Prosthesis according to any one of claims 1 to 11, characterized in that the suspension means (22) consists of a metal wire.

**Patentansprüche**

1. Mittelohrprothese (16) mit einem Schallübertragungsmittel zwischem dem Trommelfell (3) und einem Bestandteil des Steigbügels (8), wobei das Übertragungsmittel einen ersten Arm (17) und einen zweiten Arm (19) umfaßt, der kürzer als der erste Arm (17) ist, dadurch gekennzeichnet, daß die beiden Arme (17, 19) die an einem ihrer Enden durch ein Verbindungselement (18) miteinander verbunden sind, in zwei zueinander parallelen Ebenen ausgerichtet und derart gestaltet sind, daß sie bei Draufsicht in einer Richtung senkrecht zu den beiden Ebenen einen spitzen Winkel miteinander bilden, daß das Verbindungselement (18) senkrecht zu den beiden Ebenen verläuft und die Arme (17, 19) an den Enden, die den Scheitel dieses spitzen Winkels bilden, verbinden, daß das Übertragungsmittel mit einem Befestigungsmittel (23) verbunden ist, und zwar mittels einer Aufhängung (22), die langgestreckt, flexibel, ausrichtbar und justierbar ist und deren eines Ende mit dem Befestigungsmittel (23) verbunden ist, während das andere Ende mit dem Schallübertragungsmittel an einer Stelle des Endes des einen der Arme (17, 19) im Bereich des Verbindungselementes (18) zwischen den beiden Armen (17, 19) verbunden ist, und daß die Prothese (16) somit derart in die Mittelohrhöhle einsetzbar ist, daß das Befestigungsmittel (23) in einen Knochenteil des Mittelohres einsetzbar ist, daß die Aufhängung (22) unabhängig vom Trommelfell (3) ist, daß der erste Arm (17) in Berührung mit dem Trommelfell (3) steht, und daß der zweite Arm (19) in Berührung mit einem Bestandteil des Steigbügels (8) steht.

2. Prothese nach Anspruch 1, dadurch gekennzeichnet, daß diese dazu geeignet ist, in die Mittelohrhöhle derart eingesetzt zu werden, daß das Befestigungsmittel (23) in den Gehörgang eingesetzt wird.

3. Prothese nach Anspruch 1, dadurch gekennzeichnet, daß diese dazu geeignet ist, derart in die Mittelohrhöhle eingesetzt zu werden, daß das Befestigungsmittel (23) in den Trommelfellrahmen (24) eingesetzt ist.

4. Prothese nach einem der Ansprüche 1-3, dadurch gekennzeichnet, daß das Verbindungselement (18) ortsfest ist.

5. Prothese nach einem der Ansprüche 1-3, dadurch gekennzeichnet, daß das Verbindungselement (18) ausrichtbar und justierbar ist.

6. Prothese nach einem der Ansprüche 1-5, dadurch gekennzeichnet, daß diese dazu geeignet ist, derart in die Mittelohrhöhle eingesetzt zu werden, daß das freie Ende seines zweiten Armes (19) mit dem Bogen (9) des Steigbügels (8) durch ein zweites Verbindungselement (20) verbunden ist, wodurch zwischen dem zweiten Arm (19) und dem Bogen (9) ein Winkel von annähernd 90° gebildet wird.

7. Prothese nach einem der Ansprüche 1-5, dadurch gekennzeichnet, daß diese u.a. eine Stütze (21) umfaßt, die mit dem zweiten Arm (19) durch ein zweites Verbindungselement verbunden ist, das mit dem ersten Arm (19) einen Winkel von annähernd 90° bildet, und daß die Stutze (21) dazu geeignet ist, mittels ihres freien Endes die Berührung mit dem Plättchen (10) des Steigbügels (8) herzustellen, wenn die Prothese (16) in die Mittelohrhöhle eingesetzt ist.

8. Prothese nach einem der Ansprüche 6 und 7, dadurch gekennzeichnet, daß das zweite Verbindungselement (20) ortsfest ist.

9. Prothese nach einem der Ansprüche 6 und 7, dadurch gekennzeichnet, daß das zweite Verbindungselement (20) ausrichtbar und justierbar ist.

10. Prothese nach einem der vorausgegangenen Ansprüche, dadurch gekennzeichnet, daß das Befestigungsmittel (23) bei einem Knochenteil des Mittelohres und das Schallübertragungsmittel aus bio-verträglichem Keramik bestehen.

11. Prothese nach Anspruch 10, dadurch gekennzeichnet, daß das Befestigungsmittel (23) bei einem Knochenteil des Mittelohres und das Schallübertragungsmittel aus bioverträglichem, bio-aktiven Keramik bestehen.

12. Prothese nach einem der vorausgegangenen Ansprüche, dadurch gekennzeichnet, daß die Aufhängung (22) aus bioverträglichem, synthetischen Material besteht.

13. Prothese nach einem der Ansprüche 1-11, dadurch gekennzeichnet, daß die Aufhängung (22) aus einem Metalldraht besteht.

Fig. 1.

Fig. 2.

Fig. 3.

*Fig. 4 .*